(19) Europäisches Patentamt
European Patent Office
Office européen des brevets

(11) **EP 4 070 725 A1**

(12) **EUROPEAN PATENT APPLICATION**

(43) Date of publication:
**12.10.2022 Bulletin 2022/41**

(21) Application number: **21167241.5**

(22) Date of filing: **07.04.2021**

(51) International Patent Classification (IPC):
**A61B 5/11** $^{(2006.01)}$ **G16H 50/20** $^{(2018.01)}$

(52) Cooperative Patent Classification (CPC):
**A61B 5/1102; A61B 5/4878; G16H 50/20; G16H 50/50;** G16H 40/63

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DELLIMORE, Kiran Hamilton J.**
**5656 AE Eindhoven (NL)**

• **BULUT, Murtaza**
**5656 AE Eindhoven (NL)**
• **VAN LIESHOUT, Ron Martinus Laurentius**
**5656 AE Eindhoven (NL)**
• **BOUMA, Peter Hermanus**
**5656 AE Eindhoven (NL)**

(74) Representative: **Philips Intellectual Property & Standards**
**High Tech Campus 52**
**5656 AG Eindhoven (NL)**

(54) **PREDICTING BALLISTIC FORCES ON A SUBJECT**

(57) According to an aspect, there is provided an apparatus for predicting a ballistic force on a subject, the apparatus comprising: a memory comprising instruction data representing a set of instructions; and a processor configured to communicate with the memory and to execute the set of instructions, wherein the set of instructions, when executed by the processor, cause the processor to: model the body of the subject as a mechanical system comprising a plurality of masses joined by springs and dampers, wherein each of the masses represents a different region of the body of the subject, and predict the ballistic force on the subject, based on oscillations of the mechanical system.

Fig. 3

**Description**

FIELD OF THE INVENTION

The disclosure herein relates to methods and apparatus for predicting ballistic forces on a subject. Some embodiments relate to predicting ballistocardiogram measurements. Some embodiments relate to using predicted ballistocardiogram measurements to determine internal fluid accumulation such as internal bleeding.

BACKGROUND OF THE INVENTION

Hemorrhage is a major problem in postoperative care, critical/emergency care, and acute care settings and in stroke patients. In particular, timely and confident diagnosis of internal hemorrhage at the bedside remains an important challenge across multiple settings.

When a healthy person loses more than 1000 ml of blood, the body will respond by increasing the heart rate (HR) and diastolic blood pressure (BP), and by decreasing systolic BP to maintain a stable cardiac output. However, patients on medications (e.g., beta blockers), those suffering from neuropathy (e.g., due to diabetes), etc. may have a compromised ability to compensate for blood loss and might present with only limited changes in vital signs. Moreover, younger patients have a larger compensation mechanism for blood volume fluctuation and thus the vital sign changes associated with blood loss will often occur later.

When a sustained change of vital signs is clinically observed, the confirmation of internal hemorrhage is carried out using X-rays, computerised tomography (CT) scan or ultrasound (US).

Symptoms and sequelae of hemorrhage relate to perfusion of tissues, loss of less than, or equal to, 15% of blood volume (compensated shock) may not be associated with any change in vital signs. As a result, typically hemorrhage remains undiagnosed until a significant quantity of blood loss has taken place. Definitive radiological confirmation further adds to the delay. It is also worth mentioning that transporting certain patients, such as those who have undergone major surgery is not without risks, further increasing the need for improved confidence in diagnosing internal hemorrhage at the bedside.

To summarize, bedside warning systems for diagnosis rely on measures of HR and BP, but these vitals are not specific for internal hemorrhage and may even be insensitive for certain patient groups (e.g., those on beta blockers). There is a need for more specific and sensitive indicators for detecting hypovolemic shock (also known as hemorrhagic shock, or internal bleeding).

SUMMARY OF THE INVENTION

As described above, there is a need for improved methods of diagnosing and monitoring of internal bleeds. To this end, some studies have focussed on the use of Ballistocardiogram (BCG) measurements to diagnose internal bleeding as the Ballistocardiogram signal changes as the blood volume decreases. Thus, a changing Ballistocardiogram measurement can indicate an internal bleed.

One element of this technique, however, is that it requires a baseline measurement to be performed, which in some clinical (e.g. emergency) situations may be very challenging or even impossible to obtain. For example, if a patient is subject to monitoring commencing from an advanced stage of fluid accumulation, then without a baseline, these methods cannot be used to assess the patient's condition. Rather, such methods would only be able to detect whether there is further development from this initial condition. This therefore imposes a limitation on the application of such methods to clinical situations such as postoperative internal bleeding monitoring in which it is feasible and facile to obtain a baseline measurement.

The need for a baseline measurement stems from the fact that the BCG signal depends on several factors which vary from person to person (i.e., inter-person variability) and within a given individual over time (i.e., intra-person variability). The main factors influencing inter-person variability include age, anatomy, average heart rate, body mass index (BMI), gender, health conditions, posture, and weight. In contrast, intra-person variability is influenced by factors such as natural physiological processes (e.g., menstruation), chronic pathological conditions (e.g., Chronic obstructive pulmonary disease, COPD - which can cause fluid accumulation in the lungs and ankles), weight loss or gain, and heart abnormalities (e.g., arrhythmias). These processes typically have a different time scale compared to internal bleeding.

In previous work it was found that inter-person variability in BCG measurements is more prominent than intra-person variability. Significant variation from person to person is observed, with one of the strongest factors identified as gender. In contrast, measurements taken on the same individual within one week were found to be relatively stable and repeatable. Personal baseline values were found to be consistent across the two Test Rounds (which were up to 1 week apart) with a paired-sample t-test p-value (i.e., probability value) of 0.757. Since this p-value is close to 1, it indicates that there is almost no difference between the baseline datasets from the two rounds.

In summary it would be highly advantageous to account for both inter-personal and intra-personal factors and in so doing eliminate the need for a baseline measurement when detecting fluid accumulation using ballistocardiogram measurements.

Thus, according to a first aspect herein there is an apparatus for predicting a ballistic force on a subject. The apparatus comprises a memory comprising instruction data representing a set of instructions; and a processor configured to communicate with the memory and to execute the set of instructions. The set of instructions, when executed by the processor, cause the processor to: model the body of the subject as a mechanical system comprising a plurality of masses joined by springs and dampers, wherein each of the masses represents a different region of the body of the subject, and predict the ballistic force on the subject, based on oscillations of the mechanical system.

According to a second aspect there is a patient monitor comprising the apparatus of the first aspect.

According to a third aspect there is a computer implemented method for predicting a ballistic force on a subject, the method comprising: modelling the body of the subject as a mechanical system comprising a plurality of masses joined by springs and dampers, wherein each of the masses represents a region of the body of the subject, and predicting the ballistic force on the subject, based on oscillations of the mechanical system.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the third aspect.

Thus embodiments herein allow ballistic forces acting on a subject's body, such as ballistocardiographic forces due to the heart, to be predicted using a mechanical model. The predictions may be used in a variety of circumstances, but one application herein is to detect fluid accumulation due to, for example, internal bleeding. By comparing a measured ballistocardiogram measurement to a predicted ballistocardiogram measurement, an internal bleed may be detected in a quick and unobtrusive manner. These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

    Fig. 1 shows an apparatus according to some embodiments herein;
    Fig. 2 shows a method according to some embodiments herein;
    Fig. 3 shows an example mechanical model of the body according to some embodiments herein;
    Fig. 4 shows an example force profile of a force applied to simulate a ballistocardiographic force according to some embodiments herein;
    Figs. 5a-5d show the model of Fig 3 a) without internal bleeding, b) with fluid intake c) after the patient has eaten and d) in the presence of internal bleeding;
    Figs. 6a and 6b show another method according to some embodiments herein;
    Fig. 7 shows a method of placing sensors according to some embodiments herein; and
    Fig. 8 shows a further method according to some embodiments herein.

DETAILED DESCRIPTION OF EMBODIMENTS

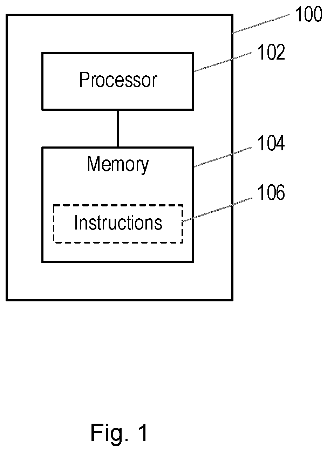

Turning now to Fig. 1 in some embodiments there is an apparatus 100 for use in predicting a ballistic force on a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 200 as described below.

Embodiments of the apparatus 100 may be for use in predicting a ballistic force on a subject. More specifically, the set of instructions, when executed by the processor, cause the processor to: model the body of the subject as a mechanical system comprising a plurality of masses joined by springs and dampers, wherein each of the masses represents a different region of the body of the subject, and predict the ballistic force on the subject, based on oscillations of the mechanical system.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively, or in addition, one or more memories 104 may be external to (i.e. separate to or remote from) the apparatus 100. For example, one or more memories 104 may be part of another device. Memory 104 can be used to store the model, physical characteristics of the subject, the predicted ballistic force and/or any other information or data received, calculated or determined by the processor 102 of the apparatus 100 or from any interfaces, memories or devices that are external to the apparatus 100. The processor 102 may be configured to control the memory 104 to store the model, physical characteristics of the subject, the predicted ballistic force.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, there is a patient monitor, comprising the apparatus 100. Such a monitor may, for example, be a bedside monitor. A monitor may comprise one or more sensors for monitoring a subject/patient. Sensors may include, for example a Ballistocardiograph (BCG) sensor.

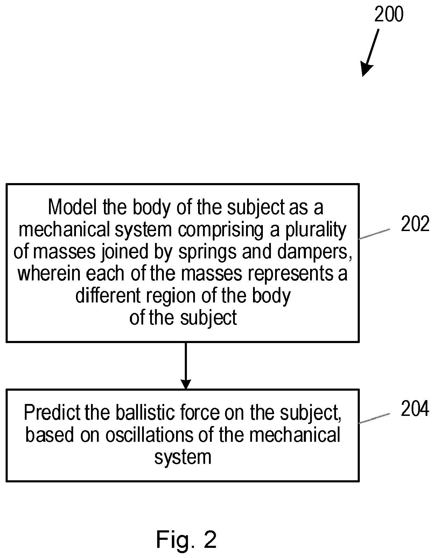

Turning to Fig. 2, there is a computer implemented method 200 for use in for predicting a ballistic force on a subject. Embodiments of the method 200 may be performed, for example by an apparatus such as the apparatus 100 described above.

Briefly, in a first step 202, the method 200 comprises: modelling the body of the subject as a mechanical system comprising a plurality of masses joined by springs and dampers, wherein each of the masses represents a region of the body of the subject. In a second step 204 the method 200 then comprises predicting the ballistic force on the subject, based on oscillations of the mechanical system.

In this manner, ballistic forces, such as ballistocardiogram measurements, can be predicted for a subject using a mechanical model. As will be described below, experiments have shown that the methods herein have high accuracy and are able to predict baseline (e.g. normal) ballistocardiogram measurements for an individual as well as ballistocardiogram measurements in the presence of a hemorrhage, and/or other factors such as intake of fluid or solids (e.g. food) which also have a known effect on ballistocardiogram measurements. As will be described below, the predicted measurements may be used in a wide variety of circumstances, for example, in determining fluid accumulation in a patient due internal bleeding. The method allows a "normal" ballistocardiogram measurements to be predicted for an individual, and this may be used as a "baseline" with which to compare against actual measured ballistocardiogram measurements. Differences between the predicted baseline and the measured ballistocardiogram indicate internal bleeding. Thus, the methods and apparatus described herein may be used to detect internal bleeding in a reliable, non-intrusive manner, without the need for x-ray, CT scans etc.

In more detail, the subject herein may be a patient, e.g. a patient under observation at a clinical facility such as a hospital or surgery environment. Generally, the subject may be any human or animal subject, e.g. infant, child, adult or animal.

In more detail, a ballistic force is a recoil force due to an applied force, or impact on the subject.

In some examples the ballistic force is a ballistiocardiogram (ballistiocardiographic force) which measures the body displacement or impact on the body due to the heart-beat of the subject (e.g. due to the force applied to eject blood from the heart during a heart-beat). Thus in some embodiments, the method 200 is for predicting a ballistiocardiogram (BCG) measurement due to the heart-beat of the subject.

More generally, a ballistic force may be the response of the body to any applied force, however. Other examples include, but are not limited to, ballistic forces as a result of processes that are external to the human body, for example, due to an external vibration source e.g. due to motions of a vehicle.

In step 202 of the method 200, the body of the subject is modelled (e.g. using a computerimplemented model) as a mechanical system comprising a plurality of masses joined by springs and dampers. In the model, each of the masses represents a part of the body of the subject. The masses may be lumped masses.

The models herein may be modelled using any suitable simulation software such as, for example, *COMSOL*™, Matlab™. Elmer™, OpenFOAM™. SimScale™.

In embodiments herein, a region (e.g. element or component) of the body comprise one or more body parts. Examples of regions include but are not limited to, the upper torso, the lower torso, and the lower extremities (e.g. legs and hips). Regions may comprise one or more body parts including but not limited to one or more organs, limbs and/or the head. A region of the body may also be used herein to refer to a particular volume e.g. within in the body, such as a volume in the lower torso that is accumulating fluid, e.g. during a hemorrhage.

The model may be a biodynamic (mass-dashpot-spring) mathematical model of the human body. In some embodiments, the model is a (simplified) four degree of freedom (4DOF) lumped mechanical system biodynamic model.

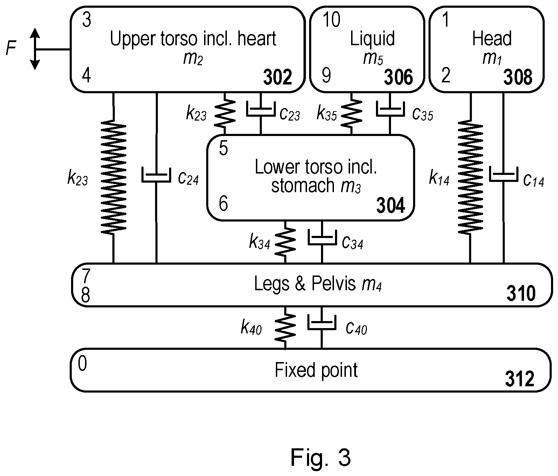

An example model is shown in Fig. 3. In the embodiment shown in Fig. 3, the plurality of masses are: a mass for the upper torso including the heart 302, a mass for the lower torso including the stomach 304, a mass for the head 308, a mass representing liquid 306, and a mass representing the legs and pelvis 310. The model is grounded at a fixed point 312. In the example in Fig. 3, the Liquid 306 represents a fluid accumulation in the lower torso. Liquid 306 is thus attached to and interacts with the mass representing the lower torso 304.

It will be appreciated that the model in Fig. 3 is an example only and that different combinations of masses may be used to model the body of the subject, depending on the desired accuracy of the model. For example, mass 310 may be split into three separate masses, representing the pelvis and each leg separately.

The model may be personalised to an individual subject. For example, the sum of the masses, $m_i$, may be set proportional to the subject's body mass M (which may be obtained or measured). In other words, $\sum m_i = M_{subject}$.

In other examples, $\sum m_i = alpha(t) * Msubject$, may be used where alpha(t) is a time dependent factor to account for micro-fluctuations in the weight.

The body mass (M) can refer to lean body mass, or weight of fat. In other words, M is a measure corresponding to mass or weight, but depending on which body regions are modelled its calculation can be adapted to include different mass related measures.

Furthermore, physical characteristics of the subject may be used to distribute the subject's mass between the plurality of masses in the mechanical system and/or to set the spring characteristics (e.g. such as values of spring constants) and/or damping characteristics (e.g. such as values of damping constants) of the model. The springs and dampers may also be modelled as a mathematical function (instead of as a constant).

Example physical characteristics include but are not limited to: average heart rate, body mass index (BMI), weight, body dimensions (e.g. such as height, the dimensions of the upper and lower torso, head, distance between shoulder blades, waist, length of legs, circumference of legs etc.), age, gender and/or body composition of the subject.

As an example, the measured mass of the subject may be distributed between the plurality of masses in the model according to empirically derived mass distribution ratios, e.g. derived from anthropometric measurements from a reference population of subjects. Different ratios may be derived and used for different genders, ages, heights, nationalities etc. Such empirically derived mass distribution ratios may be found in academic literature, or derived through experiments.

It is generally noted that age and body composition are related to a change in the mentioned parameters. In addition, parameters related to natural physiological processes or pathological conditions may also be accounted for in a biodynamic model.

Spring characteristics and/or damping characteristics may also be derived empirically, based on observed body mechanics. For example, spring and damping constants may be obtained from academic literature. They tend to scale e.g. with body mass and BMI.

In some embodiments a function may be used that translates physiological characteristics to model parameters. Such a function may be based on physiological data (weight, dimensions, BMI, etc.) from many people, from which appropriate model parameters can be learnt for use. Model parameters can be adapted until a best match is achieved. Once these learning are established (e.g. experimentally in this manner) a database of physiological parameters (input) and the corresponding model parameters (output) is constructed. This may be viewed as training data for the model.

With such this training data, rule-based transformations can be used, whereby, based on physiological statistics, corresponding model parameter are determined. Something similar to a look up table or decision tree, or any other suitable mathematical function.

In another approach, using the input (physiological parameter) and output (mechanical model) parameters as training data, a machine learning ML model can be trained to estimate (e.g. predict) model parameters from physiological parameters.

The skilled person will be familiar with ML models that can be trained to take as input physiological data and output model parameters, based on a training dataset of example input physiological data instances and ground truth model parameter outputs.

Suitable models include, but are not limited to, classification models such as neural networks or random forest models. Neural networks can be trained using known methods such as gradient descent and back propagation.

Once the initial model parameters are set for an individual, they can be further tuned by taking the input force features into account and adjusting the parameters so that the output better matches the measured output. In other words, by measuring and evaluating the response of the model to the known force-model response combinations, the initial model parameters can be further personalized.

In this way, the model may be modified to the individual subject in order to account for inter-person and intra-person variability. It is noted that by incorporating more parameters it is possible to obtain a more personalized, patient specific predicted baseline BCG measurements. However, even a fairly basic model which captures some of the inter-personal and intra-personal variation may be sufficient to replace a baseline measurement.

Turning to step 204, a ballistic force may be predicted using the model by measuring the model's response to an input force. E.g. the effect that an input force has on the motion of the masses $m_i$.

As an example, to simulate the heart-beat, a force, F, may be applied to the upper torso 302 as shown in Fig. 3. The force, F, may be used to determine the 'Time response' (e.g. the response of the body due to the beating heart). Thus, in some embodiments, predicting a ballistic force for the subject comprises simulating a ballistocardiographic effect of a heart-beat of the subject on the mechanical system by applying an oscillating force to a first mass corresponding to the upper torso.

The periodicity of the applied force, F may be set according to the measured (e.g. actual) heart-rate of the subject. The amplitude of oscillations (e.g. magnitude of the force, F) may be set based on factors such as measured (or estimated) blood flow and blood pressure in the large arteries. The amplitude of the force may also depend on the mass, BMI, etc. of the subject. In some embodiments, a mathematical model may be used, taking into account physical characteristics of the body, such as the physical characteristics described above. An example of predicting force of blood flow from the heart may be found in the paper by Wiard et al. (2009) entitled: *"Estimation of Central Aortic Forces in the Ballistocardiogram under Rest and Exercise Conditions"*.

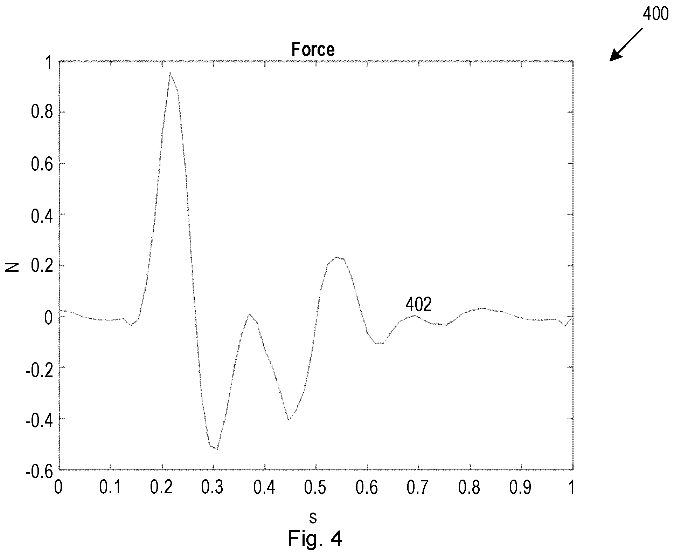

Fig. 4 illustrates a graph 400 showing an example input force profile 402 due to heartbeat that can be used as an input to the model.

As noted above, the method herein is not restricted to ballistocardiogram forces. For example, the ballistic force due to external vibrations might be measured using an input oscillating force to any other part of the body, such as for example, the legs and Pelvis (e.g. to simulate the effect on the body if the subject is standing). Another location that might be used to simulate the effect of the subject standing is the shoulder/shoulder blades. The skilled person will appreciate that this is an example only and that other vibrations on other body parts may also be simulated by applying a force to the corresponding mass in the model.

Other force profiles may also be applied (e.g. instead of oscillatory forces), for example, impulsive force profiles, or noise like (covering broader range of frequencies), or combination of these, depending on the scenario that is being simulated.

The ballistic force is predicted for a particular body part, e.g. as would be measured at a particular body part, based on the acceleration observed in the equivalent mass in the model. For example, the predicted ballistic force measurement is predicted for a particular (referred to herein as "second") region of the body, based on an acceleration of a respective second mass of the plurality of masses due to the applied oscillating force, where the second mass represents the second region of the body in the mechanical system. In this way, for example, the ballistic force as measured in the legs, head, chest or any other body part may be predicted.

The method 200 may further comprise determine an eigenfrequency of the mechanical system. The skilled person will be familiar with eigenfrequencies (or resonant frequencies). The eigenfrequency can be calculated from the mechanical properties of the model in a known manner. With respect to Fig. 3, the eigenfrequency may be determined whilst F=0, e.g. in the absence of an input force profile.

The eigenfrequency may be used set a frequency range within which to predict the ballistic force measurement from oscillations of the mechanical system (e.g. as a result of the applied oscillating force.) In some embodiments, the eigenfrequency may be used to set a cut-off frequency for the signal analysis. Typical eigenfrequencies are around 4 Hz.

In embodiments where the method is for predicting a baseline or "normal" ballistiocardiogram measurement for the subject, the predicted ballistiocardiogram may be used to determine fluid accumulation in the body. Fluid accumulation may occur, for example, in the lower torso (or other site) due to internal bleeding (hemorrhage), pleural effusion (in the upper torso e.g. lungs), and/or ascites (abnormal build-up of fluid in the abdomen or lower torso).

Fluid accumulation may generally be determined (e.g. diagnosed or predicted) by comparing the predicted ballistiocardiogram value (e.g. in the absence of fluid accumulation) to what is actually measured on the patient. Differences in what is expected compared to what is measured may indicate fluid accumulation. In other words, in some embodiments, the method 200 may further comprise obtaining a ballistocardiogram measurement of the subject, e.g. from a sensor. The method may then comprise determining whether there is internal bleeding based on a comparison of the ballistocardiogram measurement to the predicted ballistocardiogram measurement.

For example, the method 200 may further comprise determining that fluid accumulation has occurred if the measured ballistocardiogram measurement is different to the predicted ballistocardiogram measurement. For example, the measured ballistiocardiogram may be less that what would be predicted in the presence of internal bleeding, due to the lower blood volume circulating in the body.

Generally, if the difference is more than a predefined threshold then this may indicate fluid accumulation. The predefined threshold may be set, for example, based on natural (normal) variability of BCG measurements. In embodiments where the method is performed by a system such as a patient monitoring device, an alarm may be triggered, or other indication provided to a caregiver, indicating that a possible fluid accumulation has been detected.

In this way, fluid accumulation, such as hemorrhage can be detected, in a non-intrusive manner, from BCG measurements without knowledge of a baseline BCG measurement for the patient.

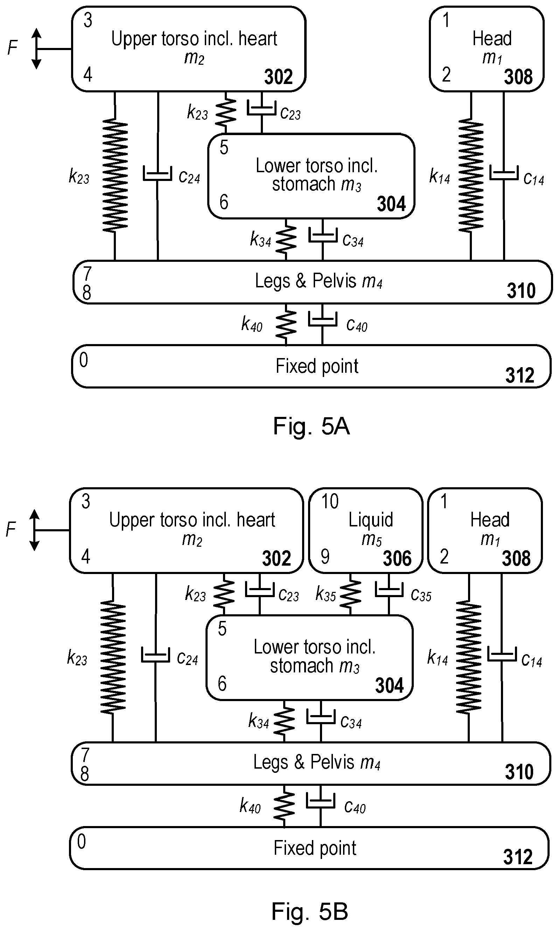

The method 200 may also be used to predict BCG measurements in other scenarios. For example, Fig, 5, shows three different such scenarios. Fig. 5a illustrates the base-line scenario shown in Fig. 3 above.

Fig. 5b shows how the baseline of Fig. 5a can be modified to simulate, liquid intake (e.g. the change to the system due to the subject having a drink). This may be modelled as an additional mass, m5, 306 connected to m3 304 with a spring and a damper. In this scenario the total mass is increased by $m_5$, the mass of the imbibed liquid.

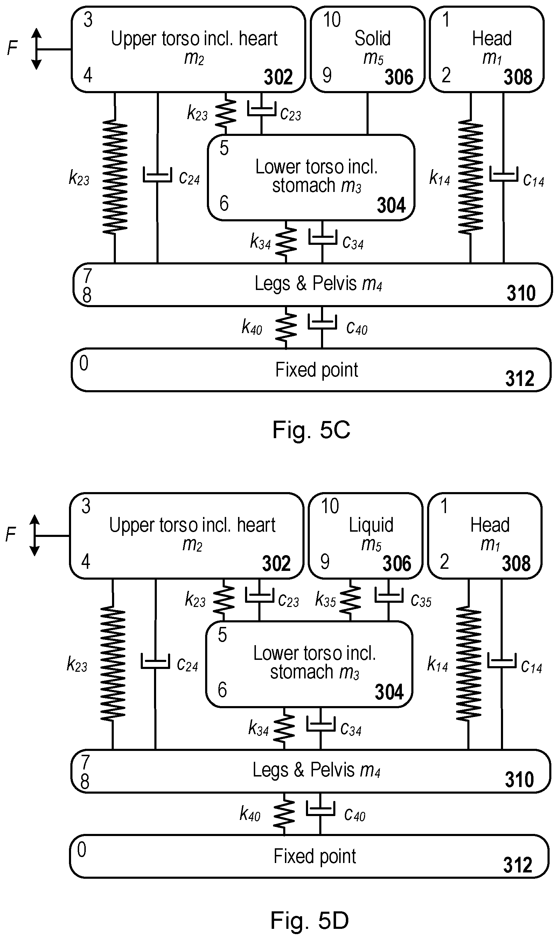

Fig. 5c shows how the baseline of Fig. 5a can be modified to simulate solid intake (e.g. food). This may be modelled as an additional mass, $m_5$, connected to m3 with a rigid connection (in essence, a mass is added to m3). In this scenario the total mass is increased by $m_5$, the mass of the consumed food.

Fig. 5d shows how the baseline of Fig. 5a can be modified to simulate internal bleeding. This may be modelled as an additional mass, m5, connected to m3 with a spring and a damper. Fig. 5d shows a model for simulating internal bleeding in the lower torso (hence the mass $m_5$ is attached to the lower torso in this example), however it will be appreciated that the mass could equally be attached to any of the masses of the model to simulate fluid accumulation in the respective body part for the connected mass. The difference in this scenario compared to eating or drinking is that mass from the other masses in the system are reduced to keep the total body mass constant. In other words, internal bleeding or other fluid accumulation represents a redistribution of mass to the respective body part (as opposed to an addition of mass).

Thus, the predicted ballistic force measurement may be predicted for the subject in the presence of fluid accumulation in the body by redistributing mass corresponding to the fluid accumulation to a mass representing the body part accumulating the fluid (e.g. such as the lower torso), whilst keeping the total mass in the mechanical system proportional (or equal) to the weight of the subject.

The model in Fig. 5d may thus be used to predict BCG values of a patient undergoing hemorrhage for different volumes (and thus masses) of blood loss. Such predictions might be used to estimate blood loss volume of a patient. Such predictions might be used to predict a BCG curve showing the expected change in BCG as hemorrhage progresses. The skilled person will appreciate that these are merely examples and that the predictions may also be made for other purposes.

Turning now to another embodiment, in this embodiment, a simplified four degree of freedom (4DOF) lumped mechanical system biodynamic model is used (as was illustrated in Fig, 3 described above). The model is applied to account for 3 parameters influencing the baseline: BMI, weight and average heart rate (i.e., a subset of the abovementioned parameters that influence inter- and intra-person variability). The model in this embodiment may be modelled using any of the simulation software described above (e.g. COMSOL™ etc.) The output of the model is obtained by monitoring the displacement and acceleration of each mass node.

In one embodiment the model may be described as below:

- Each body part is connected to another body part with a spring and a damper
- Internal bleeding: mass m5 is redistributed; total mass remains the same
- Movement due to breathing not included
- Force due to heartbeat added to torso (see Fig. 4)
- Scaling laws applied:

  - Relative mass distribution remains the same: $m_i \rightarrow m_i * m_{body}/m_{ref}$
  - Force scaled by $m^{2/3}$ (relative to $L^2$)
  - Spring and damper scaled based on mass and BMI
  - Length of person depends on mass and BMI
  - Constants are relative to the cross-section area of a person

Using the biophysical model two different types of simulations can be performed:

1. Eigenfrequency analysis - depends only on the mechanics of the human body and not on the breathing and heart rate (F = 0 N). The eigenfrequency is used to determine the frequency range for the signal analysis.
2. Time response -the body response due to the beating heart is computed at rest.

In some embodiments, the proposed biophysical model may be used as follows.

- Using input parameters specific for the subject, run the model and generate a baseline feature of the target signal or target feature. For example, if the target signal is an accelerometer signal then output will be a baseline acceleration feature.
- Use it for measuring the same target signal or target features from the subject, for example using accelerometers that are placed on the subject's body.
- Compare the generated (i.e. expected feature based on biophysical model) and measured feature to determine if there is a change in feature characteristics. As a result of fluid accumulation in the body, it is expected that the energy of the acceleration signal will decrease compared to the baseline in the presence of internal bleeding (or other fluid accumulation). In this situation the energy of the acceleration signal decreases compared to the baseline generated by the biophysical model.

Using the method 200 described above, baseline BCG measurements are no longer needed, permitting fluid accumulation diagnosis to be performed faster. It also enables fluid accumulation diagnosis and monitoring in a wider array of contexts in which it is difficult or impossible to obtain a baseline measurement.

Turning now to another example. In this example, internal bleeding is determined from BCG measurements taken using sensors at different locations on the body. For example, by comparing obtained readings of sensors at first and second locations on the body of a subject. BCG measurements at the first location might be (relatively) invariant (or have a lower level of correlation) to internal bleeding whilst BCG measurements at the second location might be more highly correlated with internal bleeding. By comparing measurements at the first 602 and second 604 locations, a differential 606 such as that illustrated in Fig, 6a may indicate internal bleeding.

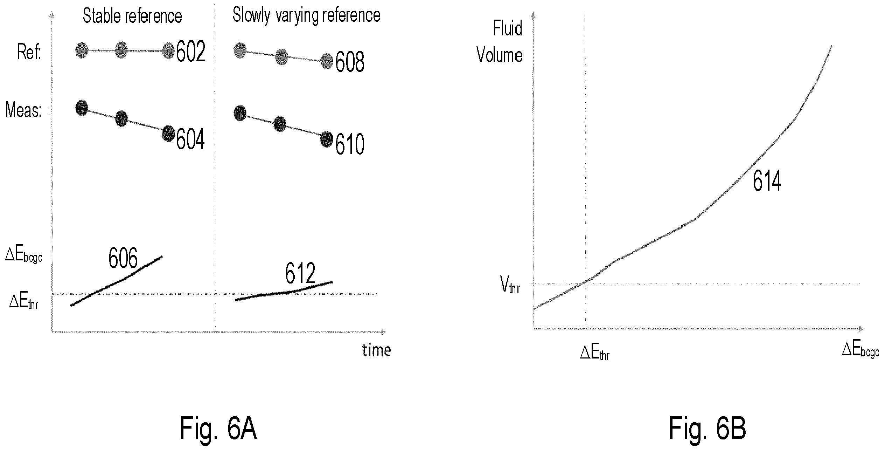

It is noted that internal bleeding may be determined from differences in the differentials of BCG measurements at different locations with respect to time. For example, measurements made at a first location where the BCG varies slowly 608 (with increased blood loss due to internal bleeding), can also indicate internal bleeding when compared to measurements made at a second location where the BCG varies more quickly 610 (with increased blood loss), for example as shown by differential 612. The line 614 in Fig. 6B shows how a differential may be correlated with fluid volume due to the internal bleeding.

There is thus provided a computer implemented method of detecting internal bleeding. Such a method may generally be performed by the apparatus 100 as described above.

In more detail, a differential measurement may be made using at least two sensors or features obtained from the same or different measurement modality, at the same or different anatomical location, in order to eliminate the need for a baseline measurement.

In general, in this embodiment, one sensor or feature may be considered a reference while the other is used as a measurement sensor or feature. The reference sensor or feature will (ideally) generate a BCG signal feature (e.g. such as energy) which is invariant or quasi-invariant to changes in fluid accumulation, while the measurement sensor or feature will generate BCG signal energy that varies significantly according to the amount of fluid accumulation present. The absolute difference between the BCG signal feature obtained from the reference and measurement sensors/features, $|\Delta E_{bcg}|$, is then computed and tracked over time to determine the fluid accumulation status of the individual. If $|\Delta E_{bcg}|$ is above a threshold value, $\Delta E_{thr}$, then this is indicative of fluid accumulation (as shown in Fig. 6). As the difference becomes larger, the more fluid is accumulated in the body.

In practice, the approach described in this embodiment may be accomplished in one of the following ways, as shown in Fig, 7:

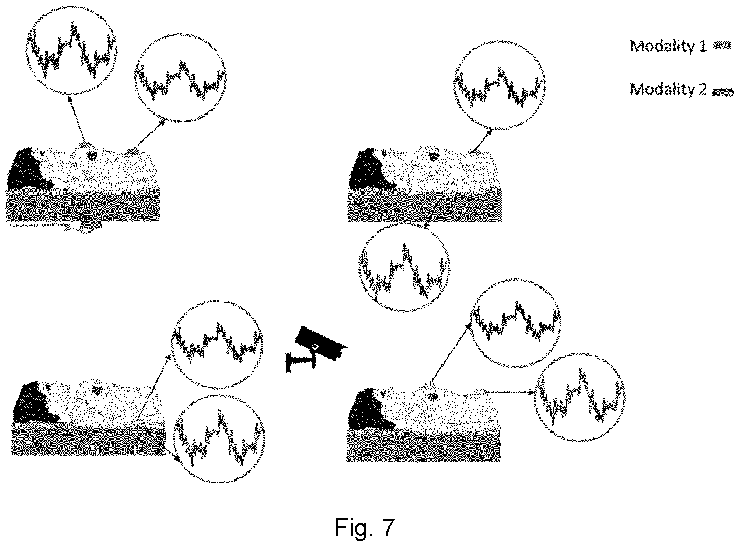

i) Same modality at different locations (Fig. 7 top left): The reference and measurement sensors are both of the same type but are positioned at different anatomical locations on the body. For example, by using two accelerometers, with the reference sensor located above the heart (where the BCG signal does not vary greatly with fluid accumulation) and the measurement sensor located at the stomach or lower back.

ii) Different modality at different locations (Fig. 7 top right): At least two different sensors at different anatomical locations: For example, by using an accelerometer and a pressure sensor (for example as part of a pressure sensitive mat) at the stomach and back locations, respectively. In this case the accelerometer is the measurement sensor and the pressure sensor serves as the reference.

iii) Different modality at the same location (Fig 7 bottom left): For example, by using an accelerometer and the pressure sensor (e.g., Early Sense, Emfit, TekScan) at the back. In this case the accelerometer may be used as the measurement sensor and the pressure sensor as the reference. The assumption here is that the pressure sensor and accelerometer have a slightly different response to the movement of the body.

iv) Same modality at multiple locations (Fig. 7 bottom right): For example, by using a camera based system it is possible to simultaneously track and acquire signals from multiple locations on the body, including at least one location which can be used a reference and at least one location which can be used for the measurement. In the case illustrated in the figure the chest is used as the reference and the stomach as the measurement.

To further underpin this embodiment, data obtained from a volunteer study suggests that the location above the heart is (quasi)invariant to changes in the BCG with fluid accumulation. In contrast the BCG signal at the stomach site was found to undergo large changes due to fluid accumulation. Based on the volunteer study it was found that various statistical measures (e.g. standard deviation, median absolute deviation) of the pressure and/or some intrinsic mode function (IMF) signals (such as the fourth IMF, for example) consistently showed poorer performance in detection of fluid accumulation. These may thus be suitable for use as a reference. The IMF is one of the decomposition signals that results from applying empirical mode decomposition (EMD) on a BCG signal.

In summary, this example is based on the fact that different measurement modalities respond differently to a fluid accumulation, and by observing the change in their difference with time, we can determine if fluid accumulation is happening or not. If with time, there is no difference between the signals this is an indication of no fluid accumulation, while any change in the inter-signal differences indicates a potential fluid accumulation, from which an alarm or alert can be generated. Similarly, the same types of sensors when placed at different body locations are expected to respond differently to the fluid accumulation, and due to this, observations of how their differences vary in time can be used to check for fluid accumulation.

Computing the difference between signals, can be performed by first calculating a residual signal, and then computing features from it, or by first computing features from each signal, and then calculating the differences between features. Note that the effect of sensor location on the measured signal can be derived from the model.

This provides a method of predicting internal bleeding using BCG measurements made on the patient (in real time) without the need for a base-line measurement. In this way, internal bleeding may thus be detected even if an internal bleed is relatively advanced.

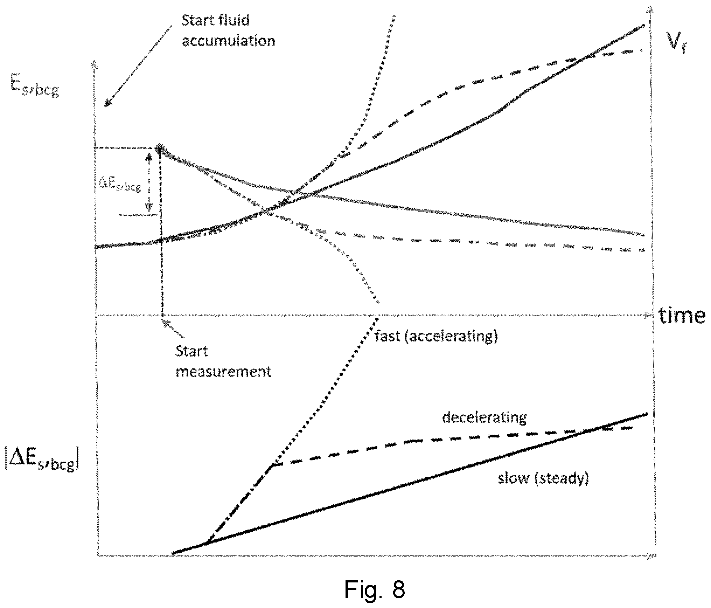

There is also another example, as shown in Fig. 8, for using trends in BCG signals to eliminate the need for a baseline measurement when determining internal bleeding. This example may be embodied in a computer implemented method and/or performed by the apparatus 100 as described above.

In this example, it is proposed to eliminate the need for a baseline measurement by using a processing sub-unit which analyses temporal trends in the BCG signal feature linked to fluid accumulation. This is based on the assumption that there is an inverse relation between fluid accumulation volume and BCG signal energy:

$$V_f(t) \propto 1/E_{s,BCG}(t) \qquad\qquad\qquad (1)$$

Therefore, as more fluid accumulates the BCG signal energy decreases. The absolute change in the signal energy over time, $|\Delta E_{s,bcg}(t)|$, relative to the initial BCG measurement provides insight into the progression of fluid accumulation in the body. This is illustrated in Fig. 8, in which the fluid accumulation changes are tracked temporally for three different fluid accumulation scenarios. A fast, accelerating fluid accumulation (dotted line), decelerating fluid accumulation (dashed lines) and slow, steady fluid accumulation (solid line).

In a further refinement of this embodiment, it is also possible to determine the rate of fluid accumulation in the body based on the rate of change of the BCG signal energy. This is clinically important, as it enables, for example, bleeding rates to be derived which permits clinicians to make better informed decisions. For instance, a surgeon can use the bleeding rate trend over a finite period of time to decide whether to re-operate on a patient if the internal bleeding rate is fast and increasing over time or to not operate if it is slow and decreasing over time.

This embodiment uses the idea that the rate of change in the signal feature characteristics will change with time as a result of fluid accumulation. The rate of change can be easily determined by computing the derivative of the feature signal (this is a signal showing the computed feature value at a particular time). The absolute value of the derivative signal will indicate the speed of the fluid accumulation, while the derivative signal (positive, negative or zero) would indicate a potential change in the nature of the fluid accumulation.

The speed of fluid accumulation is an important parameter to determine the type of fluid accumulation and the need for action. Thus this example may be used to determine internal bleeding in a subject and also the severity of such bleeding. The mechanical model described above with respect to the method 200 may be used to validate such computations. Moreover, the model can be also used to evaluate the importance of the observed changes. Thus the mechanical model and the empirical methods described herein may be performed concurrently to further improve prediction of internal fluid accumulation.

The mechanical model 200 may be used in conjunction with the methods described with respect to Figs. 6, 7 and/or 8 in various ways. For example, the mechanical model may be used:

- To determine which of these measurement based (empirical) techniques would be suitable.
- For validation of the empirical techniques, and/or for determining confidence levels in their output.
- In the embodiment described with respect to Fig. 6: to determine appropriate locations of sensors, and/or the type of the sensors (based on the signal-to-noise-ratio, and sensor characteristics)
- to determine appropriate timings of measurements for the empirical techniques (e.g. when the computations should be performed, using how much data, etc.)

In addition, the methods in the empirical embodiments may also be used as a means for collecting relevant/training data, which can be later used to set or fine tune the mechanical model parameters (e.g. how masses should be distributed, the spring and damper constants, how different masses should be connected). In the grand scale, such data can be also useful to select the mechanical model (if there are more than one option available).

Also, one or more of the empirical embodiments described with respect to Figs 6-8 and the mechanical model described with respect to Figs 1-5 can be performed concurrently and thus be considered as individual "sensors" (or data points) from which a final output can be determined by combining the output from these. For instance, an example case would be to evaluate the confidence of each output, and select the most reliable output at a given time. In another case, the selection of which monitoring technique to use can be dependent on the patient condition. As an example a mechanical modelling approach may be applied until internal fluid accumulation is detected, after which one or both of the empirical methods may be employed for further monitoring.

Turning now to other embodiments, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computerexecutable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus for predicting a ballistic force on a subject, the apparatus comprising:

a memory comprising instruction data representing a set of instructions; and
a processor configured to communicate with the memory and to execute the set of instructions, wherein the set of instructions, when executed by the processor, cause the processor to:

model the body of the subject as a mechanical system comprising a plurality of masses joined by springs and dampers, wherein each of the masses represents a different region of the body of the subject, and predict the ballistic force on the subject, based on oscillations of the mechanical system.

2. An apparatus as in claim 1 wherein the predicted ballistic force is a predicted ballistocardiogram measurement due to a heart-beat of the subject.

3. An apparatus as in claim 1 or 2 wherein the sum of the masses is set proportional to the subject's mass, and wherein physical characteristics of the subject are used to distribute the subject's mass between the plurality of masses in the mechanical system.

4. An apparatus as in claim 3 wherein spring characteristics associated with the springs of the mechanical system and/or damping characteristics associated with the dampers of the mechanical system are set based on the physical characteristics of the subject.

5. An apparatus as in claim 3 or 4 wherein the physical characteristics comprise: body mass index, weight, body dimensions, height, age, gender and/or body composition.

6. An apparatus as in any one of the preceding claims wherein a first one of the plurality of masses represents the upper torso of the subject and wherein the processor being caused to predict a ballistic force on the subject comprises the processor being caused to:
simulate a ballistocardiographic effect of a heart-beat of the subject on the mechanical system by applying an oscillating force to the first mass corresponding to the upper torso.

7. An apparatus as in claim 6, wherein the predicted ballistic force measurement is predicted for a second region of the body, based on an acceleration of a respective second mass of the plurality of masses due to the applied oscillating force, and wherein the second mass represents the second region of the body in the mechanical system.

8. An apparatus as in claims 6 or 7, wherein the processor is further caused to:

determine an eigenfrequency of the mechanical system; and
use the eigenfrequency to set a frequency range within which to predict the ballistic force measurement from oscillations of the mechanical system.

9. An apparatus as in any one of the preceding claims wherein the predicted ballistic force measurement is predicted for the subject in the presence of fluid accumulation in the body, by redistributing mass corresponding to the fluid accumulation to a mass representing the lower torso, whilst keeping the total mass in the mechanical system proportional to the weight of the subject.

10. An apparatus as in any one of claims 2 to 8 when dependent on claim 2 wherein the apparatus is for determining fluid accumulation in the body of the subject and wherein the processor is further configured to:

obtain a ballistocardiogram measurement of the subject; and
determine whether there is fluid accumulation based on a comparison of the ballistocardiogram measurement to the predicted ballistocardiogram measurement.

11. An apparatus as in claim 10 wherein the processor being caused to determine whether there is fluid accumulation comprises the processor being caused to:
determine that fluid accumulation has occurred if the ballistocardiogram measurement is different to the predicted ballistocardiogram measurement.

12. An apparatus as in claim 9, 10 or 11 wherein the fluid accumulation is due to internal bleeding.

13. A patient monitor comprising the apparatus of any one of the preceding claims.

**14.** A computer implemented method for predicting a ballistic force on a subject, the method comprising:

modelling the body of the subject as a mechanical system comprising a plurality of masses joined by springs and dampers, wherein each of the masses represents a region of the body of the subject, and

predicting the ballistic force on the subject, based on oscillations of the mechanical system.

**15.** A computer program product comprising a computer readable medium, the computer readable medium having computer readable code embodied therein, the computer readable code being configured such that, on execution by a suitable computer or processor, the computer or processor is caused to perform the method as claimed in claim 14.

Fig. 1

200

Model the body of the subject as a
mechanical system comprising a plurality
of masses joined by springs and dampers,
wherein each of the masses represents a
different region of the body
of the subject

202

Predict the ballistic force on the subject,
based on oscillations of the mechanical
system

204

Fig. 2

Fig. 3

Fig. 4

Fig. 5A

Fig. 5B

Fig. 5C

Fig. 5D

Fig. 6A

Fig. 6B

EP 4 070 725 A1

Fig. 7

Fig. 8

Europäisches
Patentamt

European
Patent Office

Office européen
des brevets

# EUROPEAN SEARCH REPORT

Application Number

EP 21 16 7241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NIGG BENNO M ET AL: "The effect of muscle stiffness and damping on simulated impact force peaks during running", JOURNAL OF BIOMECHANICS, vol. 32, 1 January 1999 (1999-01-01), pages 849-856, XP055841140, | 1-9, 12-15 | INV. A61B5/11 G16H50/20 |
| A | * 2. Methods, p. 850-852 * <br> * 3. Results, p. 852 * | 10,11 | |
| X | CN 107 860 594 A (UNIV SOUTHWEST JIAOTONG) 30 March 2018 (2018-03-30) | 1-9, 12-15 | |
| A | * paragraphs [0005], [0010], [0029]; claim 1; figure 2 * | 10,11 | |
| X | WO 2020/126764 A1 (KONINKLIJKE PHILIPS NV [NL]) 25 June 2020 (2020-06-25) | 1-9, 12-15 | |
| A | * page 10, line 1 - page 12, line 8 * <br> * page 24, lines 3-26 * | 10,11 | |
| X | US 2017/238847 A1 (INAN OMER T [US] ET AL) 24 August 2017 (2017-08-24) | 1-9, 12-15 | |
| A | * paragraphs [0118] - [0135] * | 10,11 | TECHNICAL FIELDS SEARCHED (IPC) |
| X | WO 2007/019546 A2 (UNIV OLD DOMINION [US]; BELFOR LEE A II [US] ET AL.) 15 February 2007 (2007-02-15) | 1-9, 12-15 | A61B G16H |
| A | * paragraphs [0008], [0022] - [0026] * | 10,11 | |

The present search report has been drawn up for all claims

| Place of search | Date of completion of the search | Examiner |
|---|---|---|
| Berlin | 15 September 2021 | Trachterna, Morten |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...........................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 7241

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2021

| Patent document cited in search report | | Publication date | Patent family member(s) | | Publication date |
|---|---|---|---|---|---|
| CN 107860594 | A | 30-03-2018 | NONE | | |
| WO 2020126764 | A1 | 25-06-2020 | NONE | | |
| US 2017238847 | A1 | 24-08-2017 | US 2017238847 A1<br>WO 2016033121 A1 | | 24-08-2017<br>03-03-2016 |
| WO 2007019546 | A2 | 15-02-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIARD et al.** *Estimation of Central Aortic Forces in the Ballistocardiogram under Rest and Exercise Conditions,* 2009